(19) Europäisches Patentamt
European Patent Office
Office européen des brevets

(11) EP 3 649 207 B1

(12) **FASCICULE DE BREVET EUROPEEN**

(45) Date de publication et mention de la délivrance du brevet:
**04.05.2022 Bulletin 2022/18**

(21) Numéro de dépôt: **18750242.2**

(22) Date de dépôt: **02.07.2018**

(51) Classification Internationale des Brevets (IPC):
***C09J 7/38*** *(2018.01)*

(52) Classification Coopérative des Brevets (CPC):
(C-Sets disponibles)
**C09J 7/38; C09J 183/04;** C08G 77/12; C08G 77/16; C08G 77/20; C09J 2401/006; C09J 2423/106; C09J 2427/006; C09J 2467/006; C09J 2475/006; C09J 2483/00 (Cont.)

(86) Numéro de dépôt international:
**PCT/FR2018/000187**

(87) Numéro de publication internationale:
**WO 2019/008238 (10.01.2019 Gazette 2019/02)**

(54) **ARTICLE ADHESIF SUR LA PEAU**

AUF HAUT HAFTENDER ARTIKEL

SKIN-ADHESIVE ITEM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.07.2017 FR 1700725**

(43) Date de publication de la demande:
**13.05.2020 Bulletin 2020/20**

(73) Titulaire: **ELKEM SILICONES France SAS**
**69003 Lyon (FR)**

(72) Inventeurs:
- **MOINE, Caroline**
  **42290 Sorbiers (FR)**
- **CROS, Gaelle**
  **69360 Ternay (FR)**

(74) Mandataire: **Garcia Escomel, Cristina**
**Elkem Silicones France SAS**
**9, rue Spécia**
**69190 Saint-Fons (FR)**

(56) Documents cités:
**US-A1- 2015 203 618**

- **Bluestarsilicones: "SILCOLEASE PA PSA 502 Technical Data Sheet", , 1 octobre 2016 (2016-10-01), XP002778924, Extrait de l'Internet: URL:https://silicones.elkem.com/EN/our_offer/Product/90060535/_/SILCOLEASE-PSA-502 [extrait le 2018-03-08]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 649 207 B1

(52) Classification Coopérative des Brevets (CPC):
(Cont.)

C-Sets
**A61L 24/046;**
**C09J 183/04**

**Description**

La présente invention concerne un article adhésif sur la peau comprenant un substrat **F** enduit de manière continue ou discontinue sur au moins une des deux faces par un adhésif silicone **Z** sensible à la pression qui a été stérilisé au moyen de rayonnement gamma et qui présente un bon un collant instantané (ou « tack ») sur la peau même après stérilisation.

Le marché mondial des dispositifs médicaux portables (« Wearable Medical Devices ») est en pleine croissance. Cette très forte croissance s'explique notamment par l'intégration galopante des technologies de communication sans fil qui ouvrent de nombreuses perspectives pour la surveillance à distance des patients et l'amélioration des prestations de soins. Avec cette croissance de la surveillance à distance et le passage aux soins ambulatoires et à domicile, de plus en plus de patients portent des dispositifs médicaux fixés sur la peau. En plus des capteurs pour une surveillance du rythme cardiaque, des appareils de stomie, les dispositifs médicaux portables incluent de nouvelles fonctionnalités avancées telles que par exemple la surveillance de l'exposition cutanée aux rayons ultraviolets (UV), le taux de glycémie, etc. Cette diversité de dispositifs dont le fonctionnement nécessite une adhésion sur la peau se cumule à l'utilisation de pansements à usage médical ou paramédical. La plupart des adhésifs actuellement utilisés pour fixer à la peau les dispositifs médicaux portables ou les pansements pour les soins des plaies ont le désavantage de s'adhérer très fortement à la peau. La perception sensorielle ressentie lors du décollage de la peau de tels adhésifs après quelques heures d'utilisation seulement est généralement assez pénible et peut même causer des dommages à la couche épidermique de la peau.

Les adhésifs sous forme de gel à base de polyuréthane ou de silicone fournissent une alternative aux adhésifs sensibles à la pression et sont moins douloureux à l'usage pour le patient. Cependant, les gels à base de polyuréthane ont des problèmes de cytotoxicité limitant leur utilisation pour un usage à contact cutané. Concernant les gels en silicone, les rayonnements ionisants utilisés pour la stérilisation de certains dispositifs médicaux ont un effet particulièrement préjudiciable notamment sur leurs propriétés d'adhésion à la peau (ou « tack »). C'est pour cette raison que les adhésifs en gel de silicone sont stérilisés à l'aide d'une technique de stérilisation par l'oxyde d'éthylène qui reste délicate dans sa mise en œuvre dans la mesure où ce gaz est toxique. De plus, ce processus de stérilisation est coûteux et impacte défavorablement les coûts de fabrication de tout dispositifs médicaux portables utilisant ce type d'adhésif.

Dans le cas d'un dispositif médical, une stérilisation se définit généralement par une probabilité théorique de présence d'un micro-organisme viable sur ce même dispositif inférieure ou égale à 1 pour $10^6$ « colony forming unit » (CFU/mL). Plusieurs normes de référence ont été établies afin de mettre en place un système de contrôle strict de la stérilisation de ces dispositifs. Des exemples de technique de stérilisation sont :

- la stérilisation par rayonnement ultraviolet (UV) : Cette méthode de stérilisation est plutôt employée en recherche du fait de son faible cout de mise en œuvre. Mais son principal inconvénient est que cette méthode n'est pas antifongique, c'est à dire qu'elle ne permet pas de tuer tous les germes ;
- la stérilisation par autoclave : ce mode de stérilisation est très utilisé en milieu hospitalier pour la stérilisation des instruments chirurgicaux, textiles, pansements, verrerie, caoutchouc et certains plastiques. C'est un procédé de référence pour la stérilisation en raison notamment de sa facilité de la mise en œuvre, de son faible coût et de sa non toxicité envers l'environnement. Le principe repose sur l'utilisation de la vapeur d'eau saturée sous pression comme stérilisant qui sous l'effet de la chaleur et de l'humidité permet une destruction des contaminants par coagulation et dénaturation protéique par hydrolyse. Cependant, l'efficacité de cette stérilisation dépend des paramètres d'utilisation que sont la durée du cycle, la température, la pression et le taux d'humidité. De plus, Cette technique de stérilisation ne convient pas aux matériaux thermosensibles ou hydrosensibles et ne peut s'appliquer qu'aux matériaux polymères résistants aux hautes températures ;
- la stérilisation par traitement chimique notamment :

    o la stérilisation par une exposition du dispositif médical à l'oxyde d'éthylène : L'oxyde d'éthylène est un éther cyclique, gazeux à pression atmosphérique et à température ambiante. L'action stérilisante de ce composé est due à sa réactivité vis-à-vis des groupements aminés des acides nucléiques des microorganismes. L'inconvénient de ce procédé est que l'oxyde d'éthylène est un gaz toxique pour l'homme (cancérigène et mutagène). Par ailleurs, ce procédé nécessite d'éliminer des résidus toxiques que sont les dérivés de l'oxyde d'éthylène tels que le chlorure d'éthylène et l'éthylène glycol ; et

    o la stérilisation par plasma : des plasmas froids sont induits en soumettant un gaz (ou un mélange gazeux) à un champ électrique en opérant, soit à pression atmosphérique, soit à des pressions réduites de l'ordre de $10^{-}$ millibars qui vont générer un rayonnement UV et des espèces d'oxygènes réactives éliminant les agents pathogènes ; et

- la stérilisation par ionisation : cette technique est plus particulièrement destinée aux matériaux thermosensibles.

Cette méthode de stérilisation est largement employée à l'échelle industrielle et comporte deux variantes : la stérilisation béta et la stérilisation gamma :

◦ la stérilisation par irradiation béta (β) est généralement employée en laboratoire pour stériliser certains matériaux, cependant en raison de la forte dégradation des matériaux, elle est très peu utilisée pour la stérilisation de dispositifs médicaux, et

◦ la stérilisation par rayons gamma (γ) est largement utilisée dans le milieu médical. Un rayonnement (doses comprises entre 15 et 50 kGy) est émis par un Cobalt 60 de synthèse qui entraîne la destruction de l'ADN et de l'ARN des agents pathogènes, empêchant leur réplication et l'expression des gènes. Cette méthode présente un désavantage majeur lié au fait que certains matériaux peuvent être endommagés par ce rayonnement gamma.

Pour les dispositifs médicaux, dès lors que l'aspect contact à la peau est requis, la stérilité du matériau en contact avec la peau est un critère majeur de sécurité pour un grand nombre de dispositifs médicaux et ne peut être omis car son absence peut être lourde de conséquences. Pourtant, bien que la stérilisation gamma soit très largement utilisée dans le milieu médical, cette technique présente un chalenge majeur pour les adhésifs car après traitement ils doivent toujours permettre de maintenir fermement les dispositifs médicaux au contact de la peau tout en évitant les gênes occasionnées lors des décollages de la peau de tels adhésifs. Le contrôle des propriétés d'adhérence après stérilisation reste donc clé car on souhaite qu'elles restent stable même après ce traitement d'autant que les doses de traitement gamma utilisées pour l'industrie médicale varie de 15 à 50 kGy ce qui rend le contrôle et le maintien de la performance de l'adhésif encore plus délicat.

La demande de brevet US 2015/0203618 décrit des compositions adhésives sensibles à la pression utilisables dans le domaine du traitement des plaies qui présentent une résistance à la dégradation par rayonnement gamma. Lesdits adhésifs comprennent un copolymère avec des monomères méthacrylates et des monomères acryliques ou silicones.

Parmi les adhésifs couramment utilisés dans les dispositifs médicaux portables et qui sont au contact de la peau on peut citer les adhésifs sensibles à la pression (PSA) à base de silicone qui sont susceptibles d'adhérer à une surface simplement par contact ou sous l'effet d'une pression légère. Ils présentent des avantages importants par rapport aux adhésifs acryliques. En effet, non seulement les adhésifs acryliques peuvent provoquer une irritation de la peau chez certains patients, mais ils ont également tendance à augmenter l'adhérence de la peau au fil du temps, rendant délicat le repositionnement du dispositif médical. Les PSA en silicone sont idéalement adaptés aux besoins accrus des nouveaux dispositifs médicaux portables du fait de leur biocompatibilité et de leur perméabilité permettant la diffusion de l'oxygène, du dioxyde de carbone et de la vapeur d'eau ce qui les rend parfaitement adaptés aux applications médicales dans lesquelles une aération accrue est nécessaire. Cependant, l'adhésion à la peau doit être maintenu dans une zone de confort pour le patient afin d'éviter cette gêne sensorielle liés aux phases de décollement du dispositif médical.

Le terme "adhésifs sensibles à la pression" (PSA, de l'anglais « pressure sensitive adhesives »), tel qu'il est utilisé dans le présent mémoire concerne des adhésifs qui peuvent adhérer sur une surface et en être décollés sans qu'il y ait transfert de quantités notables d'adhésif sur la surface et qui peuvent être collés de nouveau sur la même ou sur une autre surface car l'adhésif conserve une partie ou la totalité de sa pégosité et de sa force d'adhésion.

Les compositions adhésives silicones sensibles à la pression sont évaluées en termes de propriétés d'adhésion sur la peau ou « toucher collant » (en anglais : tack) et de force de pelage en anglais : (peel). Le toucher collant caractérise l'adhésivité d'un adhésif sensible à la pression et met en jeu deux facteurs : la nature des liaisons qui se créent instantanément avec le support et la viscoélasticité de l'adhésif.

Pour apprécier et évaluer le collant instantané ou « tack », une méthode dite de « Probe Tack » est connue et est décrite dans la norme ASTM D2979. Ce test permet de mesurer l'adhésion instantanée de l'adhésif. Le principe est le suivant pour les adhésifs PSA silicones décrits dans le présent mémoire : un poinçon cylindrique à face plane est amené en contact avec le film d'adhésif qui est déposé sur le substrat. Le poinçon est maintenu ensuite en contact avec le PSA silicone pendant un temps de contact d'une seconde à une pression constante de 100 gf/$cm^2$. Ensuite, le poinçon est décollé à vitesse constante de 10 mm/s du gel, et la force nécessaire pour séparer l'adhésif de la tige est mesurée et s'exprime en gf/$cm^2$ alors que l'énergie de décollement elle s'exprime en mJ/$cm^2$. Dans le présent mémoire, lorsqu'il est fait référence à la propriété de « tack » à la peau, cette propriété est évaluée via l'énergie de décollement du PSA testé.

Le pouvoir adhésif ou pelage (« peel » en anglais) d'un adhésif silicone sur la peau est la force nécessaire pour le décoller d'une feuille de papier bristol, simulant la peau, de dimension bien définie, à un angle de 180° et à une vitesse constante de 300 mm/min et avec l'aide d'une cellule de force de 10N (newtons) dans le cas des gels silicones. Il est évalué par la méthodologie décrite le document FINAT Test Method n°1 (FINAT Technical Handbook 6th édition, 2001). Ainsi, un article adhésif à la peau ayant des dimensions définies et comprenant un support sur lequel est enduit l'adhésif silicone est appliqué en mettant en contact l'adhésif silicone sur une feuille de papier bristol. L'article est ensuite décollé et la force est mesurée et ramenée à la largeur de l'article et s'exprime en g/cm (ou N/cm).

Les adhésifs à base de silicone présentent des propriétés intéressantes pour des applications biomédicales. Malheureusement, comme mentionné ci-dessus, ils sont sensibles à la stérilisation par rayons gamma et perdent les propriétés de tack adaptées à une utilisation atraumatique sur la peau des patients.

Les auteurs de la présente invention ont résolu ce problème et trouvé de façon surprenante qu'il est à présent possible de mettre au point un article adhésif sur la peau comprenant un adhésif silicone qui après une stérilisation par rayons gamma ($\gamma$) maintient ses propriétés de tack adaptées à une utilisation sur la peau.

Ainsi, la présente invention a pour objet de proposer de nouveaux articles adhésifs à la peau répondant aux problématiques énumérées ci-dessus.

Cet objectif est atteint par l'invention qui concerne un article adhésif à la peau comprenant un substrat **F** enduit de manière continue ou discontinue sur au moins une des deux faces par un adhésif silicone **Z** sensible à la pression obtenu par réticulation d'une composition silicone **X** comprenant :

1) de 80 à 20 parties en poids d'au moins une résine silicone **A** comprenant des fonctions SiOH,
2) de 20 à 80 parties en poids d'au moins un polyorganosiloxane **G2** comprenant au moins deux fonctions Si-vinyle en bout de chaine et qui est une gomme silicone ayant une consistance à 25°C comprise entre 200 mm/10 et 2000 mm/10,
3) une base silicone **B1** apte à réagir par des réactions d'addition comprenant :

- au moins un organohydrogénosiloxane ayant au moins 2 fonctions SiH en une quantité suffisante pour fournir un ratio molaire SiH/Sivinyl compris entre 0.5 :1 et 20 :1,
- un catalyseur de la réaction d'addition **C2**, et
- éventuellement un inhibiteur de la réaction d'addition, et

4) au moins un solvant **S**,

avec la condition selon laquelle :

a) la quantité de solvant **S** est déterminée de manière à ce que la composition silicone **X** contient pondéralement en extrait sec de silicone de 20 à 80%, et de préférence de 40 à 70%, et
b) ledit article adhésif sur la peau est stérilisé au moyen de rayonnement gamma à des doses comprises entre 10 kGy et 50 kGy.

La Demanderesse a mis en œuvre d'importants moyens de recherche et de nombreuses expérimentations pour atteindre cet objectif parmi d'autres. Et au terme de cela, elle a eu le mérite de trouver, de manière tout à fait surprenante et inattendue, qu'un article enduit d'un adhésif silicone **Z** sensible à la pression ayant une composition spécifique et réticulant par des réactions de polyaddition permet d'obtenir un produit après irradiation gamma ayant une variation des propriétés de « tack » sur la peau inférieure à 5% (mesures des forces de pelages). Ceci est d'autant plus surprenant qu'il est largement répandu que les adhésifs en silicone sont particulièrement connus pour subir une altération significative de leurs propriétés physiques lors d'un traitement par rayonnement gamma (phénomène de sur-réticulation, scission des macromolécules, cyclisation, oxydation, etc.). Ce type de traitement est bien connu pour entrainer une réticulation plus poussée et une scission radicalaire des chaines des polymères résultant sur des pertes de propriétés physiques du matériau en silicone (perte de flexibilité, de la résistance à la traction ou à l'allongement et/ou une augmentation de la dureté).

La résine silicone **A** comprenant des fonctions SiOH peut-être choisie parmi les résines silicones classiques parmi lesquelles on peut citer les résines organosiliciques préparées par cohydrolyse et cocondensation de chlorosilanes choisis dans le groupe constitué de ceux de formules $(R^5)_3SiCl$, $(R^5)_2Si(Cl)_2$, $R^5Si(Cl)_3$ et $Si(Cl)_4$. Ces résines sont des oligomères ou polymères organopolysiloxanes ramifiés bien connus et disponibles dans le commerce. Elles présentent, dans leur structure, au moins deux motifs siloxyles différents choisis parmi ceux de formule $(R^5)3SiO_{1/2}$ (motif M), $(R^5)_2SiO_{2/2}$ (motif D), $R^5SiO_{3/2}$ (motif T) et $SiO_{4/2}$ (motif Q), l'un au moins de ces motifs étant un motif T ou Q. Les radicaux $R^3$ sont répartis de telle sorte que les résines comportent environ 0,8 à 1,8 radicaux $R^5$ par atome de silicium. De plus, ces résines ne sont pas complètement condensées et contiennent des groupes OH. Les radicaux $R^5$ sont identiques ou différents et sont choisis parmi les radicaux alkyles linéaires ou ramifiés en $C_1$-$C_6$, les radicaux alcényles en $C_2$ - $C_4$, phényle, trifluoro-3,3,3 propyle. On peut citer par exemple comme radicaux $R^5$ alkyles, les radicaux méthyle, éthyle, isopropyle, tertiobutyle et n-hexyle et comme radicaux alcényle les groupes vinyles ou allyles. De préférence, les radicaux $R^5$ sont des groupes méthyle, vinyle ou hydroxyle. Comme exemples de résine silicone **A** on peut citer les résines MQ, les résines MDQ, les résines DT et les résines MDT, les groupes OH pouvant être portées par les motifs M, D et/ou T, la teneur pondérale en groupes OH étant comprise généralement entre 0,2 et 10 % en poids.

Selon un mode de réalisation particulier, la résine silicone **A** comprenant des fonctions SiOH est choisie parmi

le groupe constitué par :

a) les résines silicones hydroxylées de type $MQ^{(OH)}$ qui sont des copolymères comprenant des motifs siloxy M et $Q^{(OH)}$ de formules suivantes :

- M $=R^1R^2R^3SiO_{1/2}$, et
- $Q^{(OH)} = (OH)SiO_{3/2}$,
- avec éventuellement la présence de motif siloxy Q = $SiO_{4/2}$

b) les résines silicones hydroxylées de type $MD^{Vi}Q^{(OH)}$ qui sont des copolymères comprenant des motifs siloxy M, $D^{Vi}$ et $Q^{(OH)}$ de formules suivantes :

- M $=R^1R^2R^3SiO_{1/2}$,
- $D^{Vi}= (Vi)(R^1)SiO_{2/2}$, et
- $Q^{(OH)} = (OH)SiO_{3/2}$,
- avec éventuellement la présence de motif siloxy Q= $SiO_{4/2}$

c) les résines silicones hydroxylées de type $MM^{Vi}Q^{(OH)}$ qui sont des copolymères comprenant des motifs siloxy M, $M^{Vi}$ et $Q^{(OH)}$ de formules suivantes :

- M $=R^1R^2R^3SiO_{1/2}$,
- $M^{Vi} = (Vi)(R^1)(R^2)SiO_{1/2}$, et
- $Q^{(OH)} = (OH)SiO_{3/2}$,
- avec éventuellement la présence de motif siloxy Q = $SiO_{4/2}$

d) les résines silicones hydroxylées de type $MDT^{(OH)}$ qui sont des copolymères comprenant des motifs siloxy M, D et $T^{(OH)}$ de formules suivantes :

- M $=R^1R^2R^3SiO_{1/2}$,
- D = $R^1R^2SiO_{2/2}$,
- $T^{(OH)} = (OH)R^1SiO_{2/2}$, et

e) les résines silicones hydroxylées de type $DT^{(OH)}$ qui sont des copolymères comprenant des motifs siloxy D et $T^{(OH)}$de formules suivantes :

- D = $R^1R^2SiO_{2/2}$,
- $T^{(OH)} = (OH)R^1SiO_{2/2}$,

formules dans lesquelles le symbole Vi= un groupe vinyle, les symboles $R^1$, $R^2$ et $R^3$ sont choisis indépendamment les uns des autres parmi :

- les groupes alkyles linéaires ou ramifiés ayant de 1 à 8 atomes de carbone inclus et éventuellement substitués par un ou plusieurs atomes d'halogène, et de préférence choisi parmi le groupe constitué par les groupes méthyle, éthyle, isopropyle, tertiobutyle et n-hexyle, et
- les groupes aryles ou alkylaryles ayant de 6 à 14 atomes de carbone inclus, et de préférence choisi parmi le groupe constitué par les groupes phényle, xylyle et tolyle.

Selon un mode de réalisation préférentiel, la résine silicone **A** est une résine silicone hydroxylée de type $MQ^{(OH)}$ ou $MM^{Vi}Q^{(OH)}$ et contient de 0,1 à 4 % en poids de groupe hydroxyle par rapport à la masse à sec de ladite résine silicone **A.**

Pour décrire les polyorganosiloxanes, on utilise la nomenclature connue dans le domaine des silicones et qui utilise pour décrire des motifs siloxy les lettres suivantes : M, D, T et Q. La lettre M représente l'unité monofonctionelle de formule $(R)_3SiO_{1/2}$, l'atome de silicium étant relié à un seul atome d'oxygène dans le polymère comprenant cette unité. La lettre D signifie une unité difonctionnelle $(R)_2SiO_{2/2}$ dans laquelle l'atome de silicium est relié à deux atomes d'oxygène. La lettre T représente une unité trifonctionnelle de formule $(R)SiO_{3/2}$, dans laquelle l'atome de silicium est relié à trois atomes d'oxygène. La lettre Q représente une unité trifonctionnelle de formule $SiO_{4/2}$, dans laquelle l'atome de silicium est relié à quatre atomes d'oxygène. Le symbole R a la même définition que les symboles $R^2$, $R^3$ et $R^4$ définis ci-dessous. Les motifs M, D, T peuvent être fonctionnalisés. On parle alors de motifs M, D, T tout en précisant les radicaux spécifiques.

Le polyorganosiloxane **G2** selon l'invention comprend de préférence les motifs siloxy de formules suivantes :

$$M^{Vi} = [(Vi)(R^2)_2SiO_{1/2}] \text{ et } D= [R^3R^4SiO_{2/2}]$$

formules dans lesquelles:
Vi = groupement vinyle ; $R^2$, $R^3$ et $R^4$ sont des radicaux, identiques ou différents, choisis parmi le groupe constitué par :

- les radicaux alkyles linéaires ou ramifiés en $C_1$-$C_6$ tels que par exemple méthyle, éthyle, propyle, isopropyle, butyle, isobutyle, t-butyle, n-pentyle, n-hexyle,
- les radicaux cycloalkyles en $C_3$-$C_8$ tels que par exemple cyclopentyle, cyclohexyle,
- les radicaux aryles en $C_6$-$C_{10}$ tels que par exemple phényle, naphtyle et
- les radicaux alkylaryles en $C_7$-$C_{15}$ tels que par exemple tolyle, xylyle.

De façon encore plus préférentielle, le polyorganosiloxane **G2** est un $\alpha$, $\omega$-bis(vinyl) polydimethylsiloxane ce qui signifie que les radicaux $R^2$, $R^3$ et $R^4$ des motifs siloxy du polyorganosiloxane **G2** sont des radicaux méthyles. Le polyorganosiloxane **G2** présente une viscosité dynamique comprise entre 20000 et 600000 mPa.s à 25°C, de préférence entre 100000 et 600000 mPa.s à 25°C ou une consistance comprise entre 200 et 2000 exprimée en dixièmes de millimètres à 25°C (comprise entre 200 mm/10 et 2000 mm/10). Une huile $\alpha$, $\omega$-bis(vinyl) polydiméthylsiloxane de viscosité dynamique comprise entre 20000 et 600000 mPa.s à 25°C et une gomme $\alpha$, $\omega$-bis(vinyl) polydiméthylsiloxane de consistance comprise entre 200 et 2000 exprimée en dixièmes de millimètres à 25°C sont préférentiellement utilisées en tant que polyorganosiloxane **G2**.

La viscosité dynamique des silicones est mesurée à 25°C selon la norme ASTM D 445. Le terme gomme est utilisé pour des composés organosiliciques présentant des viscosités classiquement supérieures à ~600000 mPa.s ce qui correspond à un poids moléculaire supérieur à 260000 g/mole. La consistance ou pénétrabilité d'une gomme est déterminée à 25°C au moyen d'un pénétromètre de type PNR12 ou modèle équivalent permettant d'appliquer sur l'échantillon une tête cylindrique dans des conditions normalisées. La pénétrabilité d'une gomme est la profondeur exprimée en dixième de millimètres à laquelle un cylindre calibré pénètre dans l'échantillon pendant une minute. A cet effet, un échantillon de gomme est introduit dans un godet en aluminium de diamètre 40 mm et d'une hauteur de 60 mm. La tête cylindrique en bronze ou en laiton mesure 6,35 mm de diamètre et 4,76 mm de hauteur et est portée par une tige métallique de 51 mm de long et de 3mm de diamètre qui s'adapte au pénétromètre. Cette tige est lestée d'une surcharge de 100 g. Le poids total de l'ensemble est de 151,8g dont 4,3 pour la pièce cylindrique et sa tige support. Le godet contenant l'échantillon de gomme est mis dans le bain thermostaté à 25 $\pm$ 0,5 °C pendant au minimum 30mn. La mesure est effectuée en suivant la notice du constructeur. Les valeurs de la profondeur (V) en dixième de millimètre et du temps (t) en secondes pour atteindre cette profondeur sont indiquées sur l'appareil. La pénétrabilité est égale à 60V/t exprimée en dixièmes de millimètre par minute.

Selon un mode de réalisation de l'invention, le solvant **S** est choisi parmi le groupe constitué par : les hydrocarbures aliphatiques en $C_6$ à $C_{16}$, les polydiméthylsiloxanes à terminaison triméthylsilyle ayant une viscosité de 0,65 à 5 mPa.S à 25°C, les polydiméthylsiloxanes cycliques, le 3-octyl-heptaméthyl-trisiloxane, le toluène, le xylène, un ester d'alkyle en $C_1$ à $C_8$, un acide carboxylique en $C_2$ à $C_4$ et leurs mélanges.

Comme ester d'alkyle en $C_1$ à $C_8$ préféré on peut citer l'acétate d'éthyle.

La réticulation pour obtenir l'adhésif silicone **Z** sensible à la pression est initiée en évaporant le solvant **S** de préférence en maintenant l'article adhésif sur la peau dans une enceinte au sein de laquelle la température est comprise entre 50° C et 200° C, et de préférence la température au sein de l'enceinte est maintenue à plus ou moins 5°C du point d'ébullition dudit solvant **S.**

Le substrat peut-être un support de nature très variée, selon le domaine d'application. On peut citer par exemple des supports de haute énergie de surface, tels que les métaux, l'aluminium, le verre. On peut citer aussi par exemple des matières plastiques telles que des films en polyester, en polyimide, en polyéthylène téréphtalate, ou certains polydiméthylsiloxanes.

Selon un mode de réalisation préféré, le substrat **F** est un textile tissé, non-tissé ou tricoté, ou un film de matière plastique. Par le terme « non-tissé », on entend toute structure constituée de matières textiles, comme des fibres, des filaments continus ou des fils coupés, quelle qu'en soit la nature ou l'origine, formée en voile par un moyen quelconque, et liée par tout moyen, excluant l'entrelacement de fils. Les non-tissés sont des produits ayant l'apparence de textiles, poreux, composés principalement de fibres et sont fabriqués par des procédés autres que la filature, le tissage, le tricotage ou le nouage.

Selon un autre mode de réalisation préféré, le substrat **F** est en matière plastique. Une grande variété de matières plastiques peut être appropriée pour une utilisation en tant que substrat selon l'invention. Des exemples comprennent : le polychlorure de vinyle, le polypropylène, la cellulose régénérée, le polyéthylène téréphtalate (PET) et

le polyuréthane, en particulier le polyuréthane fondu-soufflé. Le substrat **F** peut-être un film souple perforé en polyuréthane ou un film souple continu en polyuréthane. Ce film souple en polyuréthane peut être fabriqué à partir de polyuréthane fondu soufflé. Lorsque le substrat **F** est un film souple en polyuréthane l'épaisseur sera généralement comprise entre 5 et 600 $\mu$m, de préférence entre 5 à 250 $\mu$m et encore plus préférentiellement entre 10 et 100 $\mu$m.

Selon un autre mode de réalisation préféré, le substrat **F** est un film souple continu qui est perméable à l'air et imperméable aux fluides. Le film peut présenter un taux de transmission de vapeur d'eau (MVTR : « Moisture Vapor Transmission Rate ») variable suivant l'application visée. Une technique de mesure du taux de transmission de vapeur d'eau en contact liquide est décrite dans la norme NF-EN 13726-2. De préférence, le film souple en polyuréthane sera choisi de manière à obtenir un pansement ayant un taux de transmission de vapeur d'eau supérieur à 300 g/m$^2$/24 heures, de préférence supérieur ou égal à 600 g/m$^2$/24 heures, de préférence encore supérieur ou égal à 1000 g/m$^2$/24 heures. Selon une autre variante avantageuse de l'invention le film souple continu en polyuréthane est perforé de manière à pouvoir favoriser la circulation des exsudats.

Des exemples d'organohydrogénosiloxane ayant au moins 2 fonctions SiH sont des réticulants ou des allongeants comprenant :

- au moins deux motifs siloxy de formule (XL-1), et de préférence au moins trois motifs siloxy de formule (XL-1) :

$$(H)\ (L)_e\ SiO_{(3-e)/2} \qquad (XL\text{-}1)$$

  dans laquelle le symbole H représente un atome d'hydrogène, le symbole L représente un alkyle ayant de 1 à 8 atomes de carbone inclus ou un aryle en $C_6$ à $C_{10}$, et le symbole e est égal à 0, 1 ou 2 ; et
- éventuellement des autres motifs siloxy de formule (XL-2):

$$(L)_g\ SiO_{(4-g)/2} \qquad (XL\text{-}2)$$

  dans laquelle le symbole L représente un alkyle ayant de 1 à 8 atomes de carbone inclus ou un aryle en $C_6$ à $C_{10}$ et le symbole g est égal à 0, 1, 2 ou 3.

Comme exemples utiles d'organohydrogénosiloxane ayant au moins 2 fonctions SiH qui a une fonction de réticulant on peut citer ceux de formules $M^H\ D_x\ D_w^H\ M^H$, $M^H\ D_x\ D_y^H$ M et M $D_x\ D_z^H$ M, formules dans lesquelles :

- $M^H$ = motif siloxyle de formule : $(H)(CH_3)_2SiO_{1/2}$
- $D^H$ = motif siloxyle de formule : $(H)(CH_3)\ SiO_{2/2}$
- D = motif siloxyle de formule : $(CH_3)_2SiO_{2/2}$ ; et
- M = motif siloxyle de formule : $(CH_3)_3SiO_{1/2}$
- avec :

  o x est un nombre compris entre 0 et 500, de préférence entre 2 et 250, et encore plus préférentiellement entre 5 et 80,
  ◦ w est un nombre compris entre 0 et 500, de préférence compris entre 1 et 250 ou entre 1 et 100, et encore plus préférentiellement compris entre 1 et 70 ;
  ◦ y est un nombre compris entre 1 et 500, de préférence compris entre 3 et 250 ou entre 2 et 100, et encore plus préférentiellement compris entre 2 et 70 ; et
  ◦ z est un nombre compris entre 2 et 500, de préférence compris entre 3 et 250 ou entre 3 et 100, et encore plus préférentiellement compris entre 3 et 70, et
  ◦ comprenant entre 2,5 % et 15,0 % en poids de fonction Si-H par polymère, de préférence entre 3,0 % et 15,0 % en poids de fonction Si-H par polymère, et encore plus préférentiellement entre 3,5 % et 12,5 % en poids de fonction Si-H par polymère.

Comme exemple de catalyseur de la réaction d'addition **C2** utile selon l'invention, on peut citer les composés d'un métal appartenant au groupe du platine bien connu de l'homme de l'art. Les métaux du groupe du platine sont ceux connus sous le nom de platinoïdes, appellation qui regroupe, outre le platine, le ruthénium, le rhodium, le palladium, l'osmium et l'iridium. On utilise, de préférence, les composés du platine et du rhodium. On peut, en particulier, utiliser les complexes du platine et d'un produit organique décrit dans les brevets US-A-3 159 601, US-A-3 159 602, US-A-3 220 972 et les brevets européens EP-A-0 057 459, EP-A-0 188 978 et EP-A-0 190 530, les complexes du platine et d'organosiloxanes vinylés décrits dans les brevets US-A-3 419 593. Le catalyseur généralement préféré est le platine. A titre d'exemples, on peut citer le platine noir, l'acide chloroplatinique, un acide chloroplatinique modifié par un alcool, un complexe de l'acide chloroplatinique avec une oléfine, un aldéhyde, un vinylsiloxane ou un alcool acétylénique, entre

autres. La préférence va à la solution ou complexe de Karstedt, tel que décrit dans le brevet US-A-3 775 452, à l'acide chloroplatinique hexahydrate ou un catalyseur au platine comprenant des ligands carbènes.

Comme exemple d'inhibiteur de la réaction d'addition utile selon l'invention, on peut citer celui choisi parmi les alcools $\alpha$-acétyléniques, les diesters $\alpha$-$\alpha$'-acétyléniques, les composés conjugués ène-yne, les cétones $\alpha$-acétyléniques, les acrylonitriles, les maléates, les fumarates et les mélanges de ceux-ci. Ces composés capables de remplir la fonction d'inhibiteur d'hydrosilylation sont bien connus de l'homme du métier. Ils peuvent être utilisés seuls ou en mélanges.

Un inhibiteur de type alcool $\alpha$-acétylénique peut être choisi parmi les composés de formule (D1) suivante :

$$(R^1)(R^2)C(OH)\text{-}C{\equiv}CH \qquad (D1)$$

dans laquelle :

- le groupe $R^1$ représente un groupe alkyle, un groupe cycloalkyle, un groupe (cycloalkyl)alkyle, un groupe aryle en $C_6$ à $C_{10}$ ou un groupe arylalkyle en $C_7$ à $C_{18}$,
- le groupe $R^2$ représente un atome d'hydrogène, un groupe alkyle, un groupe cycloalkyle, un groupe (cycloalkyl)alkyle, un groupe aryle en $C_6$ à $C_{10}$ ou un groupe arylalkyle en $C_7$ à $C_{18}$,
- ou bien $R^1$ et $R^2$ constituent ensemble avec l'atome de carbone auquel ils sont liés un cycle aliphatique à 5, 6, 7 ou 8 chaînons, éventuellement substitué une ou plusieurs fois.

Selon la formule (D1) :

- par « alkyle », on entend une chaîne hydrocarbonée saturée contenant de 1 à 20 atomes de carbone, de préférence de 1 à 8 atomes de carbone. Un groupe alkyle peut être choisi dans le groupe constitué par méthyle, éthyle, isopropyle, n-propyle, tert-butyle, isobutyle, n-butyle, n-pentyle, isoamyle et 1,1-diméthylpropyle ;
- par « cycloalkyle », on entend selon l'invention un groupe hydrocarboné saturé monocyclique ou polycyclique, de préférence monocyclique ou bi cyclique, contenant de 3 à 20 atomes de carbone, de préférence de 5 à 8 atomes de carbone. Lorsque le groupe cycloalkyle est polycyclique, les multiples noyaux cycliques peuvent être rattachés les uns aux autres par une liaison covalente et/ou par un atome spinanique et/ou être condensés les uns aux autres. Un groupe cycloalkyle peut être choisi dans le groupe constitué par le cyclopropyle, le cyclobutyle, le cyclopentyle, le cyclohexyle, le cycloheptyle, le cyclooctyle, l'adamantane et le norborane ;
- par « (cycloalkyl)alkyle », on entend selon l'invention un groupe cycloalkyle tel que défini ci-avant lié à un groupe alkyle tel que défini ci-avant également ;
- Par « aryle », on entend selon l'invention un groupe hydrocarboné aromatique contenant de 6 à 10 atomes de carbone, monocyclique ou polycyclique. Un groupe aryle peut être choisi dans le groupe constitué par phényle, naphtyle et anthracényle;
- par « arylalkyle », on entend selon l'invention un groupe aryle tel que défini ci-avant lié à un groupe alkyle tel que défini ci-avant également.

Selon un mode de réalisation préféré, dans la formule (D1) $R^1$ et $R^2$ constituent ensemble avec l'atome de carbone auquel ils sont liés un cycle aliphatique non substitué à 5, 6, 7 ou 8 chaînons. Selon un autre mode de réalisation préféré, $R^1$ et $R^2$, identiques ou différents, représentent indépendamment l'un de l'autre un groupe alkyle monovalent en $C_1$ à $C_{12}$, de préférence en $C_1$ à $C_6$.

Un inhibiteur qui est un alcool $\alpha$-acétylénique utile selon l'invention peut être choisi dans le groupe constitué par les composés suivants : 1-éthynyl-1-cyclopentanol ; 1-éthynyl-1-cyclohexanol (aussi appelé ECH) ; 1-éthynyl-1-cycloheptanol ; 1-éthynyl-1-cyclooctanol ; 3-méthyl-1-butyn-3-ol (aussi appelé MBT) ; 3-méthyl-1-pentyn-3-ol ; 3-méthyl-1-hexyn-3-ol ; 3-méthyl-1-heptyn-3-ol ; 3-méthyl-1-octyn-3-ol ; 3-méthyl-1-nonyn-3-ol ; 3-méthyl-1-decyn-3-ol ; 3-méthyl-1-dodecyn-3-ol ; 3-méthyl-1-pentadecyn-3-ol ; 3-éthyl-1-pentyn-3-ol ; 3-éthyl-1-hexyn-3-ol ; 3-éthyl-1-heptyn-3-ol ; 3,5-diméthyl-1-hexyn-3-ol ; 3-isobutyl-5-méthyl-1-hexyn-3-ol ; 3,4,4-triméthyl-1-pentyn-3-ol ; 3-éthyl-5-méthyl-1-heptyn-3-ol ; 3,6-diéthyl-1-nonyn-3-ol; 3,7,11-triméthyl-1-dodecyn-3-ol (aussi appelé TMDDO) ; 1,1-diphényl-2-propyn-1-ol ; 3-butyn-2-ol ; 1-pentyn-3-ol ; 1-hexyn-3-ol ; 1-heptyn-3-ol ; 5-méthyl-1-hexyn-3-ol ; 4-éthyl-1-octyn-3-ol et 9-éthynyl-9-fluorenol.

Un inhibiteur de type diester $\alpha$-$\alpha$'-acétylénique peut être choisi parmi les composés de formule (D2) suivante :

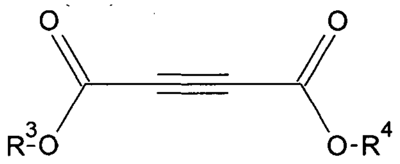

(D2)

dans laquelle les groupe $R^3$ et $R^4$, identiques ou différents, représentent indépendamment l'un de l'autre un groupe alkyle, un groupe cycloalkyle, un groupe (cycloalkyl)alkyle, un groupe aryle en $C_6$ à $C_{10}$, un groupe arylalkyle en $C_7$ à $C_{18}$ ou un groupe silyle.

Par « silyle », on entend selon l'invention un groupe de formule $-SiR_3$, dans laquel chaque symbole R représente indépendamment un groupe alkyle contenant de 1 à 20 atomes de carbone, de préférence de 1 à 8 atomes de carbone. Un groupe silyle peut être par exemple le groupe triméthylsilyle.

Selon un mode de réalisation particulier, dans la formule (D2) $R^3$ et $R^4$, identiques ou différents, représentent indépendamment l'un de l'autre un groupe alkyle en $C_1$ à $C_{12}$, de préférence en $C_1$ à $C_6$, ou le groupe triméthylsilyle. Un inhibiteur qui est un diester $\alpha$-$\alpha$'-acétylénique utile selon l'invention peut être choisi dans le groupe constitué par les composés suivants : l'acétylène-dicarboxylate de diméthyle (DMAD), l'acétylène-dicarboxylate de diéthyle, l'acétylène-dicarboxylate de tert-butyle et l'acétylène-dicarboxylate de bis(triméthylsilyle).

Un inhibiteur de type composé conjugué ène-yne peut être choisi parmi les composés de formule (D3) suivante :

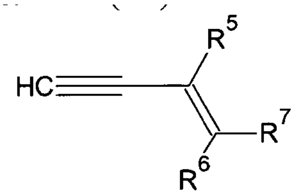

(D3)

dans laquelle :

- les groupes $R^5$, $R^6$ et $R^7$ représentent, indépendamment les uns des autres, un atome d'hydrogène, un groupe alkyle, un groupe cycloalkyle, un groupe (cycloalkyl)alkyle, un groupe aryle en $C_6$ à $C_{10}$ ou un groupe arylalkyle en $C_7$ à $C_{18}$,
- ou bien au moins deux groupes parmi les groupes $R^5$, $R^6$ et $R^7$ constituent ensemble avec le ou les atomes de carbone auxquels ils sont liés un cycle aliphatique à 5, 6, 7 ou 8 chaînons, éventuellement substitué une ou plusieurs fois.

Selon un mode de réalisation particulier, les groupes $R^5$, $R^6$ et $R^7$ représentent, indépendamment les uns des autres, un atome d'hydrogène, un groupe alkyle en $C_1$ à $C_{12}$, de préférence en $C_1$ à $C_6$, ou un groupe aryle en $C_6$ à $C_{10}$. Un inhibiteur qui est un composé conjugué ène-yne utile selon l'invention peut être choisi dans le groupe constitué par les composés suivants : le 3-méthyl-3-pentène-1-yne ; le 3-méthyl-3-hexène-1-yne ; le 2,5-diméthyl-3-hexène-1-yne ; le 3-éthyl-3-butène-1-yne ; et le 3-phényl-3-butène-1-yne. Selon un autre mode de réalisation particulier, deux groupes choisis parmi les groupes $R^5$, $R^6$ et $R^7$ constituent ensemble avec le ou les atomes de carbone auxquels ils sont liés un cycle aliphatique non substitué à 5, 6, 7 ou 8 chaînons et le troisième groupe restant représente un atome d'hydrogène ou un groupe alkyle en $C_1$ à $C_{12}$, de préférence en $C_1$ à $C_6$. Un inhibiteur qui est un composé conjugué ène-yne utile selon l'invention peut être le 1-éthynyl-1-cyclohexène.

Un inhibiteur de type cétone $\alpha$-acétylénique peut être choisi parmi les composés de formule (D4) suivante :

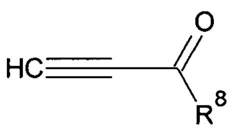

(D4)

dans laquelle : $R^8$ représente un groupe alkyle, un groupe cycloalkyle, un groupe (cycloalkyl)alkyle, un groupe aryle en $C_6$ à $C_{10}$ ou un groupe arylalkyle en $C_7$ à $C_{18}$, les groupes alkyles, cycloalkyles, (cycloalkyl)alkyle, aryle ou arylalkyle pouvant éventuellement être substitué une ou plusieurs fois par un atome de chlore, de brome ou d'iode.

Selon un mode de réalisation préféré, $R^8$ représente un groupe alkyle monovalent en $C_1$ à $C_{12}$, de préférence

en $C_1$ à $C_6$, éventuellement être substitué une ou plusieurs fois par un atome de chlore ou de brome, ou un groupe cycloalkyle, ou un groupe aryle en $C_6$ à $C_{10}$. Un inhibiteur qui est une cétone $\alpha$-acétylénique utile selon l'invention peut être choisi dans le groupe constitué par les composés suivants : la 1-octyn-3-one, la 8-chloro-1-octyn-3-one ; la 8-bromo-1-octyn-3-one ; la 4,4-diméthyl-1-octyn-3-one ; la 7-chloro-1-heptyn-3-one ; la 1-hexyn-3-one ; la 1-pentyn-3-one ; la 4-méthyl-1-pentyn-3-one ; la 4,4-diméthyl-1-pentyn-3-one ; la 1-cyclohexyl-1-propyn-3-one ; le benzoacétylène et le o-chlorobenzoyl-acétylène.

Un inhibiteur de type acrylonitrile peut être choisi parmi les composés de formule (D5) suivante :

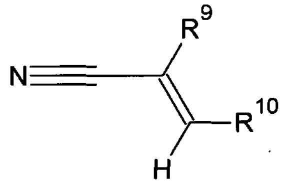

(D5)

dans laquelle : $R^9$ et $R^{10}$ représentent, indépendamment l'un de l'autre, un atome d'hydrogène, un atome de chlore, de brome ou d'iode, un groupe alkyle, un groupe cycloalkyle, un groupe (cycloalkyl)alkyle, un groupe aryle en $C_6$ à $C_{10}$ ou un groupe arylalkyle en $C_7$ à $C_{18}$, les groupes alkyles, cycloalkyles, (cycloalkyl)alkyle, aryle ou arylalkyle pouvant éventuellement être substitué une ou plusieurs fois par un atome de chlore, de brome ou d'iode.

Un inhibiteur qui est un acrylonitrile utile selon l'invention peut être choisi dans le groupe constitué par les composés suivants : l'acrylonitrile ; le méthacrylonitrile ; le 2-chloroacrylonitryle ; le crotononitrile et le cinnamonitrile.

Un inhibiteur de type maléate ou fumarate peut être choisi parmi les composés de formules (D6) et (D7) suivantes :

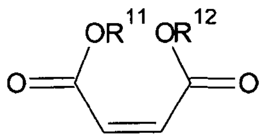

(D6)

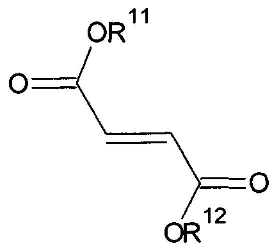

(D7)

dans lesquelles: $R^{11}$ et $R^{12}$, identiques ou différents, représentent indépendamment l'un de l'autre un groupe alkyle ou alcényle, un groupe cycloalkyle, un groupe (cycloalkyl)alkyle, un groupe aryle en $C_6$ à $C_{10}$ ou un groupe arylalkyle en $C_7$ à $C_{18}$, lesdits groupes alkyles, alcényles, cycloalkyles, (cycloalkyl)alkyles, aryles et arylalkyles pouvant être substitués par un groupe alcoxy

Par le terme « alcényle », on entend selon l'invention une chaîne hydrocarbonée saturée contenant de de 2 à 6 atomes de carbone et comprenant au moins une double insaturation. De préférence, le groupe alcényle est choisi dans le groupe constitué par un vinyle ou un allyle. Par « alcoxy », on entend selon les formules (D6) ou (D7), un groupe alkyle tel que défini ci-avant lié à un atome d'oxygène. Un groupe alcoxy peut être choisi dans le groupe constitué par méthoxy, éthoxy, propoxy et butoxy.

Selon un mode de réalisation particulier, $R^{11}$ et $R^{12}$, identiques ou différents, représentent indépendamment l'un de l'autre un groupe alkyle ou alcényle en $C_1$ à $C_{12}$, de préférence en $C_1$ à $C_6$, éventuellement substitué par un groupe alcoxy en $C_1$ à $C_6$.

Un inhibiteur qui est un maléate ou un fumarate utile selon l'invention peut être choisi dans le groupe constitué par le fumarate de diéthyle, le maléate de diéthyle, le fumarate de diallyle, le maléate de diallyle et le maléate de bis-(méthoxyisopropyle).

Ces inhibiteurs sont ajoutés en quantité en poids comprise entre 1 et 50 000 ppm par rapport au poids de la composition silicone totale, notamment entre 10 et 10 000 ppm, de préférence entre 20 et 2000 ppm et encore plus préférentiellement entre 20 ppm et 500 ppm.

Selon un mode de réalisation de l'invention, l'article adhésif à la peau selon l'invention est caractérisé en ce qu'il est un composant d'un pansement à usage médical ou paramédical.

Selon un autre mode de réalisation de l'invention, l'article adhésif à la peau selon l'invention est caractérisé en ce qu'il est un composant d'un dispositif médical portable.

Comme technique pour déposer la composition silicone **X**, on peut citer par exemple les techniques d'enduction

réalisées par racle, en particulier par racle sur cylindre, racle en l'air et racle sur tapis, ou par foulardage, c'est-à-dire par exprimage entre deux rouleaux, ou encore par rouleau lécheur, cadre rotatif, rouleau inverse "reverse roll" ou transfert, ou par pulvérisation. Comme autre technique d'enduction on peut citer la technique de couchage au rideau. Le couchage au rideau est un procédé d'application d'un liquide de couchage sur un article ou un support. Le couchage au rideau est caractérisé par la formation d'un rideau tombant librement d'un liquide de couchage qui tombe de la lèvre du Hopper et, sous l'effet de la gravité, vient rencontrer l'article se déplaçant à travers le rideau pour former un couchage (ou une enduction). Cette technique a été très utilisée dans le domaine de la préparation de supports argentiques photosensibles multicouches (voir par exemple les brevets US-3508947, US3508947 ou EP537086).

Les compositions silicones selon la présente invention possèdent de très intéressantes propriétés et qualités. Elles sont réticulables en un adhésif sensible à la pression ce qui justifie leur utilisation pour la préparation de compositions adhésives silicones sensibles à la pression. Pour leur part, les compositions adhésives silicones sensibles à la pression selon l'invention sont douées notamment simultanément de remarquables propriétés de toucher collant (en anglais : tack) et de force de pelage en anglais : (peel).

Les principales propriétés des compositions PSA sont en effet le toucher collant (tack) et la force de pelage (peel). Le toucher collant caractérise l'adhésivité d'un adhésif sensible à la pression et met en jeu deux facteurs : la nature des liaisons qui se créent instantanément avec le support et la viscoélasticité de l'adhésif. La force de pelage permet d'évaluer la force nécessaire pour séparer un ruban adhésif de son support.

Ces propriétés sont illustrées ci-après dans la partie expérimentale. Elles justifient l'utilisation des compositions silicones réticulables ci-dessus décrites, dans le revêtement total ou partiel d'une surface d'un support pour lui conférer des propriétés adhésives, ainsi que dans un procédé pour conférer des propriétés adhésives à un support dans lequel on revêt totalement ou partiellement une surface dudit support, avec une composition silicone réticulable ci-dessus décrite, puis procède à la réticulation, en évaporant le solvant, de préférence en chauffant le support siliconé à une température permettant le départ du solvant.

Les exemples qui suivent illustrent la présente demande.

Protocoles des tests

1. Toucher collant :
Norme ASTM D 2979 « Probe Tack ». Une tige métallique reliée à un capteur de force est amenée au contact d'un échantillon avec une vitesse de 1 cm/s. Le temps de contact est d'une seconde. La force nécessaire pour séparer la tige du film enduit de l'adhésif est mesurée et exprimée en $g/cm^2$.

2. Force de pelage :
Norme ASTM D 330 A. Le film enduit de l'adhésif est appliqué sur une plaque métallique ou un support de type papier (pour simuler une adhésion à la peau). Après une minute de pose, le film est décollé de la plaque sous un angle de 180° et à une vitesse constante de 300 mm/min. La force de pelage est mesurée par un capteur en g/cm.

Exemple 1 tests comparatifs

3 gels silicones commerciaux fournis par la société Bluestar Silicones ont été préparés et enduits de manière continue ou discontinue sur un film souple en polyuréthane puis stérilisés ensuite en rayonnement Gamma (à 16, 30 et 50kGy). La force de pelage des gels silicones stérilisés a été mesurée et comparée à la force de pelage sans stérilisation (les variations en % sont mentionnées dans le Tableau 1).

- Gel silicone 1: Silbione® HC2 2011
- Gel silicone 2: Silbione® HC2 2022
- Gel silicone 3: Silbione® HC2 2031

Conditions de mesure :

Mesure Pelage sur une feuille de bristol (Exacompta, dimensions : 21 cm * 5 cm)
Taille éprouvette : 2,5 cm × 14 cm ; vitesse de pelage 300 mm/min - Cellule 10 N - Pelage sur 12cm.

Tableau 1. Variations en % des forces de pelage mesurée pour les différents gels silicones

| | Variation force de pelage après traitement 16 kGy | Variation force de pelage après traitement 30 kGy | Variation force de pelage après traitement 50 kGy |
|---|---|---|---|
| Gel 1 | -47 % | -71 % | - 92% |
| Gel 2 | - 48 % | -78 % | -88 % |
| Gel 3 | - 42 % | -76 % | -97 % |

La force de pelage (sur feuille de bristol (simulant l'adhésion à la peau) des gels silicones diminue très fortement même aux faibles doses de stérilisation (16 kGy). L'adhésion à la peau de dispositifs utilisant les gels 1, 2 et 3 après stérilisation par rayonnement Gamma n'est pas satisfaisante.

Exemple 2 : tests selon l'invention

3 adhésifs silicones sensible à la pression (PSA) commerciaux fournis par la société Bluestar Silicones ont été préparés et enduits de manière continue sur un film souple en polyéthylène téréphtalate (PET) puis stérilisées ensuite en rayonnement Gamma (à 16 ou 30 kGy). La force de pelage des PSA silicones stérilisés a été mesurée et comparée à la force de pelage sans stérilisation (les variations en % sont mentionnées dans le Tableau 2).

- PSA silicone 1 : Silcolease® PSA 502 (PSA réticulant par des réactions d'addition catalysé au platine).
- PSA silicone 2 : Silcolease® PSA 400 (PSA réticulant par des réactions de condensation).
- PSA silicone 3 : Silcolease® PSA 408 (PSA réticulant par des réactions de condensation).

Conditions de mesure :

Mesure Pelage sur une feuille une plaque en acier inoxydé (fournie par la société Cheminstrument, référence TP-26 Steel panels)
Taille éprouvette : 2,5cm × 14 cm ; vitesse de pelage 300 mm/min - Cellule 100 N - Pelage sur 12 cm.

Tableau 2. Variations en % des forces de pelage mesurée pour les différents PSA silicones

| | Variation force de pelage après traitement 16 kGy | Variation force pelage après traitement 30 kGy |
|---|---|---|
| PSA silicone 1 (Invention) | -4 % | -3 % |
| PSA silicone 2 (Comparatif 1) | -14 % | -17 % |
| PSA silicone 3 (Comparatif 2) | -19 % | -11 % |

Les meilleurs résultats sont obtenus avec le PSA 1 qui est un adhésif sensible à la pression réticulant par des réactions d'addition (catalyseur platine). On remarque que la force de pelage du PSA silicone 1 après irradiation est diminuée de 3 à 4% par rapport au même PSA non irradié illustrant un changement dans la structure chimique du produit obtenu après irradiation.

Les PSA silicones 2 et 3 (comparatifs) qui sont des PSA réticulant par polycondensation montrent des variations des forces de pelage comprises entre -10 % et - 15% générant des problématiques d'adhésion, notamment lorsqu'il s'agit d'adhésion sur la peau.

**Revendications**

1. Article adhésif à la peau comprenant un substrat **F** enduit de manière continue ou discontinue sur au moins une des deux faces par un adhésif silicone **Z** sensible à la pression obtenu par réticulation d'une composition silicone **X** comprenant :

1) de 80 à 20 parties en poids d'au moins une résine silicone **A** comprenant des fonctions SiOH,
2) de 20 à 80 parties en poids d'au moins un polyorganosiloxane **G2** comprenant au moins deux fonctions Si-vinyle en bout de chaine et qui est une gomme silicone ayant une consistance à 25°C comprise entre 200 mm/10 et 2000 mm/10,
3) une base silicone **B1** apte à réagir par des réactions d'addition comprenant :

- au moins un organohydrogénosiloxane ayant au moins 2 fonctions SiH en une quantité suffisante pour fournir un ratio molaire SiH/Sivinyl compris entre 0.5:1 et 20:1,
- un catalyseur de la réaction d'addition **C2**, et
- éventuellement un inhibiteur de la réaction d'addition, et

4) au moins un solvant **S**,

avec la condition selon laquelle :

a) la quantité de solvant **S** est déterminée de manière à ce que la composition silicone **X** contient pondéralement en extrait sec de silicone de 20 à 80%, et de préférence de 40 à 70%, et
b) ledit article adhésif sur la peau est stérilisé au moyen de rayonnement gamma à des doses comprises entre 10 kGy et 50 kGy.

**2.** Article adhésif à la peau selon la revendication 1 dans lequel la réticulation pour obtenir l'adhésif silicone **Z** sensible à la pression est initiée en évaporant le solvant **S** de préférence en maintenant l'article adhésif sur la peau dans une enceinte au sein de laquelle la température est comprise entre 50°C et 200° C, et de préférence la température au sein de l'enceinte est maintenue à plus ou moins 5°C du point d'ébullition dudit solvant **S**.

**3.** Article adhésif à la peau selon l'une quelconque des revendications 1 et 2 dans lequel le solvant **S** est choisi parmi le groupe constitué par : les hydrocarbures aliphatiques en $C_6$ à $C_{16}$, les polydiméthylsiloxanes à terminaison triméthylsilyle ayant une viscosité de 0,65 à 5 mPa.S à 25°C, les polydiméthylsiloxanes cycliques, le 3-octyl-heptaméthyl-trisiloxane, le toluène, le xylène, un ester d'alkyle en $C_1$ à $C_8$, un acide carboxylique en $C_2$ à $C_4$ et leurs mélanges.

**4.** Article adhésif à la peau selon la revendication 1 dans lequel la résine silicone comprenant des fonctions SiOH est choisie parmi le groupe constitué par :

a) les résines silicones hydroxylées de type $MQ^{(OH)}$ qui sont des copolymères comprenant des motifs siloxy M et $Q^{(OH)}$ de formules suivantes :

- $M = R^1R^2R^3SiO_{1/2}$, et
- $Q^{(OH)} = (OH)SiO_{3/2}$,
- avec éventuellement la présence de motif siloxy $Q = SiO_{4/2}$

b) les résines silicones hydroxylées de type $MD^{Vi}Q^{(OH)}$ qui sont des copolymères comprenant des motifs siloxy M, $D^{Vi}$ et $Q^{(OH)}$ de formules suivantes :

- $M = R^1R^2R^3SiO_{1/2}$,
- $D^{Vi} = (Vi)(R^1)SiO_{2/2}$, et
- $Q^{(OH)} = (OH)SiO_{3/2}$,
- avec éventuellement la présence de motif siloxy $Q= SiO_{4/2}$

c) les résines silicones hydroxylées de type $MM^{Vi}Q^{(OH)}$ qui sont des copolymères comprenant des motifs siloxy M, $M^{Vi}$ et $Q^{(OH)}$ de formules suivantes :

- $M = R^1R^2R^3SiO_{1/2}$,
- $M^{Vi} = (Vi)(R^1)(R^2)SiO_{1/2}$, et
- $Q^{(OH)} = (OH)SiO_{3/2}$,
- avec éventuellement la présence de motif siloxy $Q = SiO_{4/2}$

d) les résines silicones hydroxylées de type $MDT^{(OH)}$ qui sont des copolymères comprenant des motifs siloxy

M, D et $T^{(OH)}$ de formules suivantes :

- M =$R^1R^2R^3SiO_{1/2}$,
- D = $R^1R^2SiO_{2/2}$,
- $T^{(OH)}$ = $(OH)R^1SiO_{2/2}$, et

e) les résines silicones hydroxylées de type $DT^{(OH)}$ qui sont des copolymères comprenant des motifs siloxy D et $T^{(OH)}$de formules suivantes :

- D = $R^1R^2SiO_{2/2}$,
- $T^{(OH)}$ = $(OH)R^1SiO_{2/2}$,

formules dans lesquelles le symbole Vi = un groupe vinyle, les symboles $R^1$, $R^2$ et $R^3$ sont choisis indépendamment les uns des autres parmi :

- les groupes alkyles linéaires ou ramifiés ayant de 1 à 8 atomes de carbone inclus et éventuellement substitués par un ou plusieurs atomes d’halogène, et de préférence choisi parmi le groupe constitué par les groupes méthyle, éthyle, isopropyle, tertiobutyle et n-hexyle, et
- les groupes aryles ou alkylaryles ayant de 6 à 14 atomes de carbone inclus, et de préférence choisi parmi le groupe constitué par les groupes phényle, xylyle et tolyle.

5. Article adhésif à la peau selon la revendication 4 dans lequel la résine silicone **A** est une résine silicone hydroxylée de type $MQ^{(OH)}$ ou $MM^{Vi}Q^{(OH)}$ et contient de 0,1 à 4 % en poids de groupe hydroxyle par rapport à la masse à sec de ladite résine silicone **A.**

6. Article adhésif à la peau selon l’une quelconque des revendications précédentes caractérisé en qu’il est un composant d’un pansement à usage médical ou paramédical.

7. Article adhésif à la peau selon l’une quelconque des revendications précédentes caractérisé en qu’il est un composant d’un dispositif médical portable.

8. Procédé de préparation d’un article adhésif à la peau selon l’une quelconque des revendications 1 à 7, **caractérisé en ce que** :

- on enduit un substrat **F** de manière continue ou discontinue sur au moins une des deux faces d’une composition silicone **X** comprenant :

1) de 80 à 20 parties en poids d’au moins une résine silicone **A** comprenant des fonctions SiOH,
2) de 20 à 80 parties en poids d’au moins un polyorganosiloxane **G2** comprenant au moins deux fonctions Si-vinyle en bout de chaine et qui est une gomme silicone ayant une consistance à 25°C comprise entre 200 mm/10 et 2000 mm/10,
3) une base silicone **B1** apte à réagir par des réactions d’addition comprenant :

- au moins un organohydrogénosiloxane ayant au moins 2 fonctions SiH en une quantité suffisante pour fournir un ratio molaire SiH/Sivinyl compris entre 0.5:1 et 20:1,
- un catalyseur de la réaction d’addition **C2**, et
- éventuellement un inhibiteur de la réaction d’addition, et

4) au moins un solvant **S**,

avec la condition selon laquelle la quantité de solvant **S** est déterminée de manière à ce que la composition silicone **X** contient pondéralement en extrait sec de silicone de 20 à 80%, et de préférence de 40 à 70%, et
- on procède à la réticulation de ladite composition silicone **X** pour obtenir un adhésif silicone **Z** sensible à la pression, et
- on stérilise ledit article adhésif à la peau au moyen de rayonnement gamma à des doses comprises entre 10 kGy et 50 kGy.

9. Procédé de préparation d’un article adhésif à la peau selon la revendication 8, **caractérisé en ce que** la réticulation

est initiée en évaporant le solvant **S**, de préférence en maintenant l'article adhésif dans une enceinte au sein de laquelle la température est comprise entre 50°C et 200°C, et de préférence la température au sein de l'enceinte est maintenue à plus ou moins 5°C du point d'ébullition dudit solvant **S**.

**10.** Utilisation d'un article adhésif à la peau selon l'une quelconque des revendications 1 à 7, pour la fabrication d'un pansement à usage médical ou paramédical.

**11.** Pansement à usage médical ou paramédical, **caractérisé en ce que** l'un de ses composants est un article adhésif à la peau selon l'une quelconque des revendications 1 à 7.

**12.** Utilisation d'un article adhésif à la peau selon l'une quelconque des revendications 1 à 7, pour la fabrication d'un dispositif médical portable.

**13.** Dispositif médical portable, **caractérisé en ce que** l'un de ses composants est un article adhésif à la peau selon l'une quelconque des revendications 1 à 7.

## Patentansprüche

**1.** Hautklebeartikel, umfassend ein Substrat **F,** das auf eine der beiden Seiten kontinuierlich oder diskontinuierlich beschichtet ist mit einem Silikon-Haftklebstoff **Z,** erhalten durch Vernetzen einer Silikonzusammensetzung **X,** umfassend:

1) 80 bis 20 Gewichtsteile mindestens eines Silikonharzes **A** mit SiOH-Funktionen,
2) 20 bis 80 Gewichtsteile mindestens eines Polyorganosiloxans **G2** mit mindestens zwei Si-Vinyl-Funktionen am Kettenende, bei dem es sich um ein Silikongummi mit einer Konsistenz bei 25 °C zwischen 200 mm/10 und 2000 mm/10 handelt,
3) eine Silikonbasis **B1,** die durch Additionsreaktionen reagieren kann, umfassend:

- mindestens ein Organohydrosiloxan mit mindestens 2 SiH-Funktionen in einer zur Bereitstellung eines SiH/Si-Vinyl-Molverhältnisses zwischen 0,5:1 und 20:1 ausreichenden Menge,
- einen Katalysator der Additionsreaktion **C2,** and
- gegebenenfalls einen Inhibitor der Additionsreaktion, und

4) mindestens ein Lösungsmittel **S,**

mit der Maßgabe, gemäß der:

a) die Menge an Lösungsmittel **S** so bestimmt ist, dass die Silikonzusammensetzung **X** einen Feststoffgehalt von 20 bis 80 Gew.-% und vorzugsweise von 40 bis 70 Gew.-% aufweist, und
b) der Hautklebeartikel mittels Gamma-Strahlung bei Dosen zwischen 10 kGy und 50 kGy sterilisiert ist.

**2.** Hautklebeartikel nach Anspruch 1, wobei die Vernetzung zum Erhalt des Silikon-Haftklebstoff des **Z** durch Verdampfen des Lösungsmittels **S** initiiert wird, vorzugsweise unter Halten des Hautklebeartikels in einer Kammer, in der die Temperatur zwischen 50 °C und 200 °C liegt, wobei die Temperatur in der Kammer vorzugsweise bei plus oder minus 5 °C vom Siedepunkt des Lösungsmittels **S** gehalten wird.

**3.** Hautklebeartikel nach einem der Ansprüche 1 und 2, wobei das Lösungsmittel **S** aus der Gruppe bestehend aus aliphatischen $C_6$- bis $C_{16}$-Kohlenwasserstoffen, Polydimethylsiloxanen mit einer Trimethylsilyl-Endgruppe mit einer Viskosität von 0,65 bis 5 mPa.s bei 25 °C, cyclischen Polydimethylsiloxanen, (3-Octyl)-heptamethyltrisiloxan, Toluol, Xylol, einem $C_1$- bis $C_8$-Alkylester, einer $C_2$- bis $C_4$-Carbonsäure und Mischungen davon ausgewählt ist.

**4.** Hautklebeartikel nach Anspruch 1, wobei das Silikonharz mit SiOH-Funktionen ausgewählt ist aus der Gruppe bestehend aus:

a) hydroxylierten Silikonharzen vom Typ $MQ^{(OH)}$, bei denen es sich um Copolymere mit M- and $Q^{(OH)}$-Siloxyeinheiten der folgenden Formeln handelt:

- $M = R^1R^2R^3SiO_{1/2}$ und
- $Q^{(OH)} = (OH) SiO_{3/2}$,
- gegebenenfalls mit Vorliegen der Siloxyeinheit $Q = SiO_{4/2}$

b) hydroxylierten Silikonharzen vom Typ $MD^{Vi}Q^{(OH)}$, bei denen es sich um Copolymere mit M-, $D^{Vi}$- und $Q^{(OH)}$-Siloxyeinheiten der folgenden Formeln handelt:

- $M = R^1R^2R^3SiO_{1/2}$,
- $D^{Vi} = (Vi) (R^1) SiO_{2/2}$ und
- $Q^{(OH)} = (OH) SiO_{3/2}$,
- gegebenenfalls mit Vorliegen der Siloxyeinheit $Q = SiO_{4/2}$

c) hydroxylierten Silikonharzen vom Typ $MM^{Vi}Q^{(OH)}$, bei denen es sich um Copolymere mit M-, $M^{Vi}$- und $Q^{(OH)}$-Siloxyeinheiten der folgenden Formeln handelt:

- $M = R^1R^2R^3SiO_{1/2}$,
- $M^{Vi} = (Vi)(R^1)(R^2)SiO_{2/2}$ und
- $Q^{(OH)} = (OH)SiO_{3/2}$,
- gegebenenfalls mit Vorliegen der Siloxyeinheit $Q = SiO_{4/2}$

d) hydroxylierten Silikonharzen vom Typ $MDT^{(OH)}$, bei denen es sich um Copolymere mit M-, D- und $T^{(OH)}$-Siloxyeinheiten der folgenden Formeln handelt:

- $M = R^1R^2R^3SiO_{1/2}$,
- $D = R^1R^2SiO_{2/2}$,
- $T^{(OH)} = (OH)R^1SiO_{2/2}$, und

e) hydroxylierten Silikonharzen vom Typ $DT^{(OH)}$, bei denen es sich um Copolymere mit D- and $T^{(OH)}$- Siloxyeinheiten der folgenden Formeln handelt:

- $D = R^1R^2SiO_{2/2}$,
- $T^{(OH)} = (OH) R^1SiO_{2/2}$,

wobei in den Formeln das Symbol Vi = eine Phenylgruppe und die Symbole $R^1$, $R^2$ und $R^3$ unabhängig voneinander ausgewählt sind aus:

- linearen oder verzweigten Alkylgruppen mit 1 bis 8 Kohlenstoffatomen inklusive, die gegebenenfalls durch ein oder mehrere Halogenatome substituiert sind, und vorzugsweise aus der Gruppe bestehend aus Methyl-, Ethyl-, Isopropyl-, tert-Butyl- und n-Hexylgruppen, und
- Aryl- oder Alkylarylgruppen mit 6 bis 14 Kohlenstoffatomen inklusive, vorzugsweise aus der Gruppe bestehend aus Phenyl-, Xylyl- und Tolylgruppen.

**5.** Hautklebeartikel nach Anspruch 4, wobei das Silikonharz **A** ein hydroxyliertes Silikonharz vom Typ $MQ^{(OH)}$ oder $MM^{Vi}Q^{(OH)}$ ist und 0,1 bis 4 Gew.-% Hydroxylgruppen, bezogen auf die Trockenmasse des Silikonharzes **A,** enthält.

**6.** Hautklebeartikel nach einem der vorhergehenden Ansprüche, **dadurch gekennzeichnet, dass** es sich um eine Komponente eines Wundverbands zur medizinischen oder paramedizinischen Verwendung handelt.

**7.** Hautklebeartikel nach einem der vorhergehenden Ansprüche, **dadurch gekennzeichnet, dass** es sich um eine Komponente einer tragbaren medizinischen Vorrichtung handelt.

**8.** Verfahren zur Herstellung eines Hautklebeartikels nach einem der Ansprüche 1 bis 7, **dadurch gekennzeichnet, dass**:

- man ein Substrat **F** kontinuierlich oder diskontinuierlich auf mindestens einer der beiden Seiten mit einer Silikonzusammensetzung **X** beschichtet, die Folgendes umfasst:

1) 80 bis 20 Gewichtsteile mindestens eines Silikonharzes **A** mit SiOH-Funktionen,

2) 20 bis 80 Gewichtsteile mindestens eines Polyorganosiloxans **G2** mit mindestens zwei Si-Vinyl-Funktionen am Kettenende, bei dem es sich um ein Silikongummi mit einer Konsistenz bei 25 °C zwischen 200 mm/10 und 2000 mm/10 handelt,
3) eine Silikonbasis **B1,** die durch Additionsreaktionen reagieren kann, umfassend:

- mindestens ein Organohydrosiloxan mit mindestens 2 SiH-Funktionen in einer zur Bereitstellung eines SiH/Si-Vinyl-Molverhältnisses zwischen 0,5:1 und 20:1 ausreichenden Menge,
- einen Katalysator der Additionsreaktion **C2,** und
- gegebenenfalls einen Inhibitor der Additionsreaktion, und

4) mindestens ein Lösungsmittel **S,**

mit der Maßgabe, gemäß der die Menge an Lösungsmittel **S** so bestimmt ist, dass die Silikonzusammensetzung **X** einen Feststoffgehalt von 20 bis 80 Gew.-% und vorzugsweise von 40 bis 70 Gew.-% aufweist, und
- man die Silikonzusammensetzung **X** zu einem Silikon-Haftklebstoff **X** vernetzt und
- man den Hautklebeartikel mittels Gamma-Strahlung bei Dosen zwischen 10 kGy und 50 kGy sterilisiert.

**9.** Verfahren zur Herstellung eines Hautklebeartikel nach Anspruch 8, **dadurch gekennzeichnet, dass** die Vernetzung durch Verdampfen des Lösungsmittels **S** initiiert wird, vorzugsweise unter Halten des Hautklebeartikels in einer Kammer, in der die Temperatur zwischen 50 °C und 200 °C liegt, wobei die Temperatur in der Kammer vorzugsweise bei plus oder minus 5 °C vom Siedepunkt des Lösungsmittels **S** gehalten wird.

**10.** Verwendung eines Hautklebeartikel nach einem der Ansprüche 1 bis 7 zur Herstellung eines Wundverbands zur medizinischen oder paramedizinischen Verwendung.

**11.** Wundverband zur medizinischen oder paramedizinischen Verwendung, **dadurch gekennzeichnet, dass** es sich bei einer seiner Komponenten um einen Hautklebeartikel nach einem der Ansprüche 1 bis 7 handelt.

**12.** Verwendung eines Hautklebeartikels nach einem der Ansprüche 1 bis 7 bei der Herstellung einer tragbaren medizinischen Vorrichtung.

**13.** Tragbare medizinische Vorrichtung, **dadurch gekennzeichnet, dass** es sich bei einer ihrer Komponenten um einen Hautklebeartikel nach einem der Ansprüche 1 bis 7 handelt.

## Claims

**1.** Skin-adhesive article comprising a substrate **F** coated, continuously or noncontinuously, on at least one of the two faces with a pressure-sensitive silicone adhesive **Z** obtained by crosslinking a silicone composition **X** comprising:

1) from 80 to 20 parts by weight of at least one silicone resin **A** comprising SiOH functional groups,
2) from 20 to 80 parts by weight of at least one polyorganosiloxane **G2** comprising at least two Si-vinyl functional groups at the chain end and which is a silicone gum having a consistency at 25°C of between 200 mm/10 and 2000 mm/10,
3) a silicone base **B1** capable of reacting by addition reactions, comprising:

- at least one organohydrosiloxane having at least two SiH functional groups in an amount sufficient to provide an SiH/Si-vinyl molar ratio of between 0.5:1 and 20:1,
- a catalyst of the addition reaction **C2,** and
- optionally an inhibitor of the addition reaction, and

4) at least one solvent **S,**

with the condition according to which:

a) the amount of solvent **S** is determined so that the silicone composition **X** contains, by weight as solid content of silicone, from 20% to 80% and preferably from 40% to 70%, and
b) said skin-adhesive article is sterilized by means of gamma radiation at doses of between 10 kGy and 50 kGy.

**2.** Skin-adhesive article according to Claim 1, in which the crosslinking to obtain the pressure-sensitive silicone adhesive **Z** is initiated by evaporating the solvent **S,** preferably while keeping the skin-adhesive article in a chamber within which the temperature is between 50°C and 200°C, and preferably the temperature within the chamber is kept at plus or minus 5°C from the boiling point of said solvent **S.**

**3.** Skin-adhesive article according to either one of Claims 1 and 2, in which the solvent **S** is chosen from the group consisting of: aliphatic $C_6$ to $C_{16}$ hydrocarbons, polydimethylsiloxanes comprising a trimethylsilyl end group having a viscosity of 0.65 to 5 mPa.s at 25°C, cyclic polydimethylsiloxanes, (3-octyl)heptamethyltrisiloxane, toluene, xylene, a $C_1$ to $C_8$ alkyl ester, a $C_2$ to $C_4$ carboxylic acid and their mixtures.

**4.** Skin-adhesive article according to Claim 1, in which the silicone resin comprising SiOH functional groups is chosen from the group consisting of:

a) hydroxlyated silicone resins of $MQ^{(OH)}$ type which are copolymers comprising M and $Q^{(OH)}$ siloxy units of following formulae:

- M = $R^1R^2R^3SiO_{1/2}$, and
- $Q^{(OH)}$ = (OH) $SiO_{3/2}$,
- with optionally the presence of siloxy unit Q = $SiO_{4/2}$

b) hydroxlyated silicone resins of $MD^{Vi}Q^{(OH)}$ type which are copolymers comprising M, $D^{Vi}$ and $Q^{(OH)}$ siloxy units of following formulae:

- M = $R^1R^2R^3SiO_{1/2}$,
- $D^{Vi}$ = (Vi) ($R^1$) $SiO_{2/2}$, and
- $Q^{(OH)}$ = (OH)$SiO_{3/2}$,
- with optionally the presence of siloxy unit Q = $SiO_{4/2}$

c) hydroxlyated silicone resins of $MM^{Vi}Q^{(OH)}$ type which are copolymers comprising M, $M^{Vi}$ and $Q^{(OH)}$ siloxy units of following formulae:

- M = $R^1R^2R^3SiO_{1/2}$,
- $M^{Vi}$ = (Vi)($R^1$)($R^2$)$SiO_{2/2}$, and
- $Q^{(OH)}$ = (OH) $SiO_{3/2}$,
- with optionally the presence of siloxy unit Q = $SiO_{4/2}$

d) hydroxlyated silicone resins of $MDT^{(OH)}$ type which are copolymers comprising M, D and $T^{(OH)}$ siloxy units of following formulae:

- M = $R^1R^2R^3SiO_{1/2}$,
- D = $R^1R^2SiO_{2/2}$,
- $T^{(OH)}$ = (OH) $R^1SiO_{2/2}$, and

e) hydroxlyated silicone resins of $DT^{(OH)}$ type which are copolymers comprising D and $T^{(OH)}$ siloxy units of following formulae:

- D = $R^1R^2SiO_{2/2}$,
- $T^{(OH)}$ = (OH)$R^1SiO_{2/2}$,

in which formulae the symbol Vi = a vinyl group and the symbols $R^1$, $R^2$ and $R^3$ are chosen, independently of one another, from:

- the linear or branched alkyl groups having from 1 to 8 carbon atoms and optionally substituted by one or more halogen atoms, and preferably chosen from the group consisting of the methyl, ethyl, isopropyl, tert-butyl and n-hexyl groups, and
- aryl or alkylaryl groups having from 6 to 14 carbon atoms inclusive, and preferably chosen from the group consisting of the phenyl, xylyl and tolyl groups.

**5.** Skin-adhesive article according to Claim 4, in which the silicone resin **A** is a hydroxylated silicone resin of $MQ^{(OH)}$ or $MM^{Vi}Q^{(OH)}$ type and contains from 0.1% to 4% by weight of hydroxyl group with respect to the dry weight of said silicone resin **A.**

**6.** Skin-adhesive article according to any one of the preceding claims, **characterized in that** it is a component of a dressing for medical or paramedical use.

**7.** Skin-adhesive article according to any one of the preceding claims, **characterized in that** it is a component of a wearable medical device.

**8.** Process for the preparation of a skin-adhesive article according to any one of Claims 1 to 7, **characterized in that**:

- a substrate **F** is coated, continuously or noncontinuously, on at least one of the two faces with a silicone composition **X** comprising:

1) from 80 to 20 parts by weight of at least one silicone resin **A** comprising SiOH functional groups,
2) from 20 to 80 parts by weight of at least one polyorganosiloxane **G2** comprising at least two Si-vinyl functional groups at the chain end and which is a silicone gum having a consistency at 25°C of between 200 mm/10 and 2000 mm/10,
3) a silicone base **B1** capable of reacting by addition reactions, comprising:

- at least one organohydrosiloxane having at least two SiH functional groups in an amount sufficient to provide an SiH/Si-vinyl molar ratio of between 0.5:1 and 20:1,
- a catalyst of the addition reaction **C2,** and
- optionally an inhibitor of the addition reaction, and

4) at least one solvent **S,**

with the condition according to which the amount of solvent **S** is determined so that the silicone composition **X** contains, by weight as solid content of silicone, from 20% to 80% and preferably from 40% to 70%, and
- said silicone composition **X** is crosslinked in order to obtain a pressure-sensitive silicone adhesive **Z,** and
- said skin-adhesive article is sterilized by means of gamma radiation at doses of between 10 kGy and 50 kGy.

**9.** Process for the preparation of a skin-adhesive article according to Claim 8, **characterized in that** the crosslinking is initiated by evaporating the solvent **S,** preferably while keeping the adhesive article in a chamber within which the temperature is between 50°C and 200°C, and preferably the temperature within the chamber is kept as plus or minus 5°C from the boiling point of said solvent **S.**

**10.** Use of a skin-adhesive article according to any one of Claims 1 to 7 in the manufacture of a dressing for medical or paramedical use.

**11.** Dressing for medical or paramedical use, **characterized in that** one of its components is a skin-adhesive article according to any one of Claims 1 to 7.

**12.** Use of a skin-adhesive article according to any one of Claims 1 to 7 in the manufacture of a wearable medical device.

**13.** Wearable medical device, **characterized in that** one of its components is a skin-adhesive article according to any one of Claims 1 to 7.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 20150203618 A
- US 3159601 A
- US 3159602 A
- US 3220972 A
- EP 0057459 A
- EP 0188978 A
- EP 0190530 A
- US 3419593 A
- US 3775452 A
- US 3508947 A
- EP 537086 A

### Littérature non-brevet citée dans la description

- FINAT Technical Handbook. 2001